# EUROPÄISCHE PATENTSCHRIFT

(11) **EP 0 812 822 B1**
(45) Veröffentlichungstag und Bekanntmachung des Hinweises auf die Patenterteilung: **28.07.1999**
(21) Anmeldenummer: 97106222.9
(22) Anmeldetag: 16.04.1997
(51) Int. Cl.: C07C 227/04

(54) **Verfahren zur Herstellung von Aminoessigsäuren mit alpha-ständigem tertiären Kohlenwasserstoffrest oder deren Nitrilen**
Process for the preparation of aminoacetic acids substituted at position alpha by a tertiary hydrocarbon chain or their nitriles
Procédé de préparation d'acides aminoacétiques ayant en position alpha une chaîne carbonée tertiaire ou de leurs nitriles

(30) Priorität: 10.06.1996 DE 19623141
(43) Veröffentlichungstag der Anmeldung: 17.12.1997
(73) Patentinhaber: HÜLS AKTIENGESELLSCHAFT, 45764 Marl (DE)
(72) Erfinder: Bauer, Frank, Dr., 51143 Köln (DE); Kleemiss, Wolfgang, Dr., 45721 Haltern (DE); Feld, Marcel, Dr., 51147 Köln (DE)

(56) Entgegenhaltungen:
- MIYAZAWA, TOSHIFUMI ET AL: "Synthesis of t-leucine" MEM. KONAN UNIV., SCI. SER. (1979), 23, 51-4 CODEN: MKOUAS;ISSN: 0452-4160, XP002044596

## Beschreibung

Die Erfindung betrifft ein Verfahren zur Herstellung von Aminoessigsäuren mit α-ständigem tertiären Kohlenwasserstoffrest oder deren Nitrilen aus den entsprechend α-substituierten Malonsäurederivaten.

Die einfachste Verbindung dieser substituierten Aminoessigsäuren ist tert.-Leucin. d.h. α-tert.Butylaminoessigsäure, das als nicht-proteinogene Aminosäure eine erhebliche Bedeutung für die Synthese von biologisch aktiven Proteinen mit besonderer Wirkung hat. Es dient weiterhin als Auxiliar für asymmetrische Synthesen (U.Schöllkopf, Pure and Applied Chem., **55** [1983], 1799). Das zu diesem Zweck benötigte enantiomerenreine tert.-Leucin kann durch kinetische Racematspaltung von N-Acyl-tert.-leucinen mit Hilfe einer spezifischen Deacylase gewonnen werden (EP-0 494 716). Weiterhin läßt sich tert.-Leucin leicht in Leucinol umwandeln, das z.B. als chirales Auxiliar für die stereoselektive Synthese von Insektiziden verwendet wird (M.L.McKennon et al., J.Org.Chem. **58** [1993], 3568). Andere Aminoessigsäuren mit α-ständigem tertiären Kohlenwasserstoffrest (d.h. höhere Homologe des tert.-Leucins) sollten in entsprechender Weise verwendbar sein.

Tert.-Leucin kann durch Strecker-Synthese aus Pivalinaldehyd (K.Ogura. Bull.Chem.Soc.Jpn. 65 [1992],2359) oder durch Ammonolyse von 2-Brom-3.3-dimethylbuttersäure (Abderhalden. Z.Phys.Chem. **228** [1934], 193) hergestellt werden. Entsprechend lassen sich Aminoessigsäuren mit anderen α-ständigen tertiären Kohlenwasserstoffresten aus entsprechenden anderen Aldehyden durch Strecker-Synthese oder aus den entsprechenden Bromcarbonsäuren durch Ammonolyse herstellen. Ein weiteres bekanntes Herstellverfahren für tert.-Leucin in der L-Form ist die enzymkatalysierte Transaminierung von 3,3-Dimethyl-2-oxobuttersäure (EP 0 248 357).

Sowohl Pivalinaldehyd als auch 2-Brom-3,3-dimethylbuttersäure und 3,3-Dimethyl-2-oxobuttersäure sind relativ kostspielige Ausgangsstoffe. Das gilt auch für die Aldehyde, aus denen Aminoessigsäuren mit anderen α-ständigen tertiären Kohlenwasserstoffresten als dem tert.-Butylrest analog dem von Pivalinaldehyd ausgehenden Verfahren hergestellt werden können. Für die Strecker-Synthese ist außerdem die sicherheitstechnisch anspruchsvolle Blausäure erforderlich. Bei der enzymkatalysierten Transaminierung sind zudem die Raum-Zeit-Ausbeuten unbefriedigend.

Nach Mem. Konan Univ. Sci. Ser. 23, 51-54 (1979) kann man bei der Herstellung von tert.-Leucin von Glycin ausgehen, wobei man zunächst die Aminogruppe schützt und dann schrittweise die tert.-Butylgruppe einführt. Bei einer anderen Synthese wird tert.-Butylmalonsäuremonomester als Zwischenprodukt gebildet, das dann mit Diphenylphosphorazidat (DPPA) und Triethylamin und danach mit konzentrierter Salzsäure zum tert.-Leucin umgesetzt wird. Beide Synthesen sind umständlich und liefern das Produkt nur in mäßigen Ausbeuten.

Es bestand daher die Aufgabe, ein Verfahren zur Herstellung von Aminoessigsäuren mit α-standigem tertiären Kohlenwasserstoffrest oder deren Nitrilen bereitzustellen. das. von wohlfeilen Ausgangsstoffen ausgehend, gute Ausbeuten und Raum-Zeit-Ausbeuten ergibt und keine sicherheitstechnisch anspruchsvollen Stoffe erfordert.

Erfindungsgemäß wird diese Aufgabe dadurch gelöst und werden Aminoessigsäuren mit α-ständigem tertiären Kohlenwasserstoffrest oder deren Nitrile vorteilhaft dadurch hergestellt, daß man in α-Stellung durch einen tertiären Kohlenwasserstoffrest substitituierte Malonsäuremonoamide oder deren Ester oder Nitrile einem Hofmannschen Abbau unterwirft.

Die Reaktion läßt sich für tert.-Leucin bei Verwendung von Natronlauge und Natriumhypochlorit durch das folgende Reaktionsschema veranschaulichen:

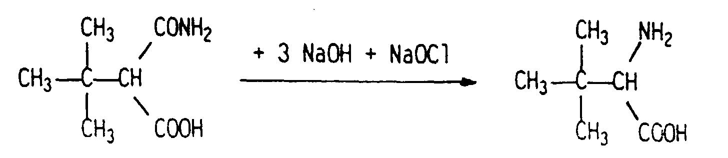

Die hervorragenden Ausbeuten des Verfahrens von deutlich mehr als 90 % der Theorie sind überraschend. Substituierte Malonsäuren neigen nämlich in alkalischem Medium dazu, durch Decarboxylierung in die entsprechenden substituierten Essigsäuren überzugehen (F.Patai. The Chemistry of Carboxylic Acids and Esters, s. 589. Interscience. New York, 1969).

Die Ausgangsstoffe für das Verfahren nach der Erfindung entsprechen im allgemeinen der Formel I 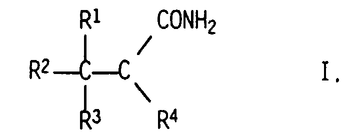 in der R¹, R² und R³ gleiche oder verschiedene Kohlenwasserstoffreste bedeuten, jeweils zwei dieser Reste mit dem quartären Kohlenstoffatom, das sie substituieren, zudem einen Kohlenwasserstoffring bilden können und R⁴ eine Carboxyl-, Carbonester- oder Nitrilgruppe bedeutet. Unter den Bedingungen des Hofmannschen Abbaus wird die Carbonestergruppe zur Carboxylgruppe verseift. Ob die Nitrilgruppe erhalten bleibt oder ebenfalls zur Carboxylgruppe verseift wird, hängt von den Reaktionsbedingungen ab. Je höher die Temperatur, je stärker die Base, je größer deren Menge und je länger die Reaktionszeit, umso mehr ist die Verseifung zur Carboxylgruppe begünstigt. Für einen gegebenen Ausgangsstoff I lassen sich die Bedingungen, unter denen man ein Reaktionsprodukt mit einer Nitril- oder mit einer Carboxylgruppe erhält, durch orientierende Versuch unschwer ermitteln.

In den bevorzugten Ausgangsstoffen I bedeuten R¹, R² und R³ gleiche oder verschiedene Alkyl-, Alkenyl-, Aryl-, Alkaryl- oder Aralkylreste mit bis zu 10 Kohlenstoffatomen und können zudem jeweils zwei dieser Reste mit dem quartären Kohlenstoffatom, das sie substituieren, einen Kohlenwasserstoffring mit 5 bis 12, inbesondere mit 5 oder 6 Kohlenstoffatomen bilden. R⁴ steht in den bevorzugten Ausgangsstoffen I für die Carboxylgruppe, die Nitrilgruppe oder eine Carbonestergruppe -COOR₅, in der R⁵ einen Alkylrest mit 1 bis 4, insbesondere mit 1 oder 2 Kohlenstoffatomen oder den Benzylrest bedeutet. Besonders bevorzugt werden Ausgangsstoffe I, in denen R⁴ eine Carboxylgruppe bezeichnet.

Den Ausgangsstoffen I entsprechend, haben die Reaktionsprodukte im allgemeinen die Formel 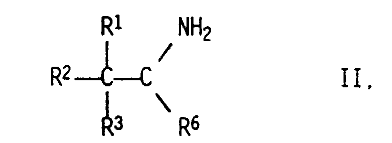 in der R¹, R² und R³ die angegebenen Bedeutungen einschließlich der bevorzugten Bedeutungen haben und R⁶ eine Carboxyl- oder eine Nitrilgruppe bedeutet.

Die Ausgangsstoffe I lassen sich in bekannter Weise herstellen, z.B. aus Cyanessigsäure oder deren Estern, indem man zunächst durch Umsetzung mit entsprechenden Elektrophilen den tertiären Kohlenwasserstoffrest einführt und dann die Nitrilgruppe zur Carbonamidgruppe verseift. Nach dem Verfahren der Erfindung lassen sich auch enantiomerenangereicherte oder enantiomerenreine Ausgangsstoffe I, die aus enantiomerenangereicherten oder enantiomerenreinen Malonsäuremonoestern mit α-ständigem tertiären Kohlenwasserstoffrest über die Zwischenstufe des Esterchlorids hergestellt werden können, zu den entsprechenden Aminoessigsäuren mit α-ständigem tertiären Kohlenwasserstoffrest umsetzen. Enantiomerenangereichertes oder enantiomerenreines tert.-Leucin läßt sich so eleganter herstellen als durch die erwähnte, vergleichsweise umständliche kinetische Racematspaltung von N-Acyl-tert.-leucinen.

Von den geeigneten Ausgangsstoffen I seien beispielsweise α-tert-Butylmalonsäuremonoamid, α-tert.-Butylmalonsäuremononitrilmonoamid (oder α-tert.-Butylcyanacetamid), α-tert.-Butylmalonsäuremoncmethylestermonoamid, α-tert.-Pentylmalonsäuremononitrilmonoamid. α-(1-Methyl-1-phenylethyl)-malonsäuremonoamid, α-(1-Methyl-1-phenylethyl)-malonsäuremonoethylestermonoamid, α-(1-Methylcyclohexyl)-malonsäuremonoamid und α-(1-Methylcyclohexyl)-malonsäuremononitrilmonoamid genannt.

Für den Hofmannschen Abbau werden eine Base und ein Salz einer unterhalogenigen Säure benötigt. vorteihaft ein Alkalihydroxid und ein Alkalihypochlorit, jeweils in Form ihrer wässerigen Lösungen. Am besten eignen sich Natronlauge und Natriumhypochlorit-Lösung, auch Bleichlauge genannt. Beide Stoffe werden in großen Mengen hergestellt und sind wohlfeile Chemikalien. Man wendet die Base zweckmäßig in Mengen von 1,5 bis 4, insbesondere von 2 bis 4 Äquivalenten je Mol Ausgangsstoff I an. Enthält dieser eine Nitrilgruppe, die verseift werden soll, so wählt man eine Basenmenge im oberen Segment des genannten Bereichs oder arbeitet mit einem noch größeren Überschuß. Das Salz der unterhalogenigen Säure wird zweckmäßig in Mengen von 1,0 bis 1,2 Äquivalenten je Mol Ausgangsstoff I verwendet.

Das Verfahren nach der Erfindung kann kontinuierlich oder diskontinuierlich durchgeführt werden. Bei einer diskontinuierlichen Ausführungsform mit Natriumhydroxid als basischem Stoff und Natriumhypochlorit als Salz einer unterhalogenigen Säure trägt man zunächst den Ausgangsstoff I in eine etwa 5- bis 40-gewichtsprozentige Natronlauge ein. Man rührt das Gemisch und setzt ihm nach und nach etwa 5 bis 15-gewichtsprozentige Natriumhypochloritlösung zu. Dabei wird die Temperatur vorteilhaft auf 0°C bis 20°C gehalten. Zur Vervollständigung der Reaktion läßt man das Reaktionsgemisch einige Zeit, z.B. 1 bis 6 Stunden, nachreagieren, wobei man es auf höhere Temperatur, z.B. bis zu 180°C erhitzt. Im allgemeinen ist die Reaktion vollständig, nachdem man das Reaktionsgemisch 1 bis 2 Stunden auf 60 bis 80°C erhitzt hat. Nach dem Abkühlen neutralisiert man das Reaktionsgemisch, zweckmäßig mit einer Mineralsäure, wie Salzsäure oder Schwefelsäure, auf einen pH-Wert von etwa 6 bis etwa 8 und erhält dadurch eine wässerige Lösung, die als Reaktionsprodukt die gewünschte substituierte Aminoessigsäure oder deren Nitril und das mineralsaure Salz enthält. Aus dieser Lösung kann man das mineralsaure Salz mittels eines der bekannten Ionenaustausch- und-/oder Membranverfahren vom Reaktionsprodukt abtrennen und die salzfreie Lösung zur Trockne einengen. Alternativ kann man das neutralisierte Reaktionsgemisch mit der substituierten Aminoessigsäure oder deren Nitril sowie dem mineralsauren Salz ohne vorhergehende Salzabtrennung zur Trockne eindampfen und den Eindampfrückstand mit einem geeigneten selektiven Lösungsmittel für die substituierte Aminoessigsäure oder deren Nitril extrahieren, z.B. mit siedendem Methanol. Dabei bleibt die Hauptmenge des mineralsauren Salzes ungelöst zurück. Wenn man aus dem Extrakt das selektive Lösungsmittel verdampft, erhält man als Rückstand eine substituierte Aminoessigsäure oder deren Nitril, die bzw. das, je nach mineralsaurem Salz und selektivem Lösungsmittel, noch 10 bis 20 Gewichtsprozent mineralsaures Salz enthalten und durch Ionenaustausch- und/oder Membranverfahren weiter gereinigt werden kann.

Der Ausgangsstoff I kann auch in situ nach dem Verfahren der Erfindung umgesetzt werden, indem man direkt von dem Reaktionsgemisch ausgeht, das bei der partiellen Verseifung der entsprechend substituierten Cyanessigsäure oder eines ihrer Ester zum Malonsäuremonoamid in basischem Medium anfällt. Man braucht dann nur noch etwa 1,5 bis 2,5 Äquivalente Base zuzusetzen und den Hofmannschen Abbau einschließlich der Aufarbeitung wie beschrieben durchzuführen.

Man kann das Verfahren nach der Erfindung auch kontinuierlich durchführen, z.B. analog der in DE-OS 44 41 777 beschriebenen Arbeitsweise. Dazu wird ein Gemisch aus Ausgangsstoff I und Alkalihydroxidlösung kontinuierlich bei 10 bis 180°C, vorteilhaft 40 bis 80°C, genügend lange mit einer Hypochloritlösung in Kontakt gebracht und das Reaktionsgemisch. wie beschrieben, kontinuierlich oder absatzweise aufgearbeitet.

Man erhält die Aminoessigsäuren mit α-ständigem tertiären Kohlenwasserstoffrest oder deren Nitrile bei allen Verfahrensvarianten mit hohen Raum-Zeit-Ausbeuten und mit Ausbeuten, die in der Regel 90 % der Theorie und mehr betragen. Durch Umkristallisieren der Produkte. z.B. aus Wasser, erhält man sie in hoher Reinheit, die z.B. bei tert.-Leucin mehr als 98 % beträgt.

### Beispiele

### Beispiel 1

In einem Rührgefäß werden 50 g (0.125 mol) 10 gewichtsprozentige Natronlauge bei Raumtemperatur vorgelegt. Man gibt 20 g (0.125 mol) α-tert.-Butylmalonsäuremonoamid zu, wobei das Gemisch gerührt und auf 10°C abgekühlt wird. Unter Rühren werden dann weitere 50 g (0.25 mol) 20 gewichtsprozentige Natronlauge zugesetzt, wobei die Temperatur auf <15°C gehalten wird. Danach dosiert man, wiederum bei einer Temperatur von <15°C, 83.8 g (0.125 mol) 11.1 gewichtsprozentige Natriumhypochloritlösung zu und läßt das Gemisch 3 Stunden bei dieser Temperatur rühren. Anschließend wird das Gemisch 3 Stunden auf 60°C erwärmt und dann auf Raumtemperatur abgekühlt. Danach wird das Reaktionsgemisch mit konzentrierter Salzsäure auf einen pH-Wert von 7 eingestellt und zur Trockne eingedampft. Der Rückstand wird mit 500 g siedendem Methanol extrahiert. Nach dem Einengen der methanolischen Lösung verbleiben 16 g tert.-Leucin, das nach Elementaranalyse noch ca. 1.5 g Natriumchlorid enthält. Die Ausbeute an tert.-Leucin unter Berücksichtigung des noch enthaltenen Natriumchlorids beträgt 98 % der Theorie.

### Beispiel 2

In einem Rührgefäß werden 160 g (0.8 mol) 20 gewichtsprozentige Natronlauge bei 50°C vorgelegt und gerührt. Man gibt 127,4 g (0,8 mol) α-tert.-Butylmalonsäuremonoamid zu. wobei das Gemisch gerührt und auf 10°C abgekühlt wird. Unter Rühren werden dann weitere 320 g (1.6 mol) 20 gewichtsprozentige Natronlauge zugesetzt. wobei die Temperatur auf <15°C gehalten wird. Danach dosiert man innerhalb von 40 Minuten und wiederum bei einer Temperatur von <15°C 536,2 g (0,8 mol) 11,1 gewichtsprozentige Natriumhypochloritlösung. Anschließend wird das Gemisch 3 Stunden auf 60°C erwärmt und dann auf Raumtemperatur abgekühlt. Dann wird das Reaktionsgemisch mit konzentrierter Salzsäure auf einen pH-Wert von 7 eingestellt und zur Trockne eingeengt. Man erhält ca. 340 g Trockenrückstand, der mit 1.000 g siedendem Methanol extrahiert wird. Nach dem Einengen der methanolischen Lösung verbleiben 100 g tert.-Leucin, das nach Elementaranalyse noch ca. 20 g Natriumchlorid enthält. Die Ausbeute an tert.-Leucin unter Berücksichtigung des noch enthaltenen Natriumchlorids beträgt 95 % der Theorie.

## Patentansprüche

1. Verfahren zur Herstellung von Aminoessigsäuren mit α-ständigem tertiären Kohlenwasserstoffrest oder deren Nitrilen, dadurch gekennzeichnet, daß man in α-Stellung durch einen tertiären Kohlenwasserstoffrest substituierte Malonsäuremonoamide oder deren Ester oder Nitrile einem Hofmannschen Abbau unterwirft.

2. Verfahren nach Anspruch 1, dadurch gekennzeichnet, daß die Ausgangsstoffe der Formel I: 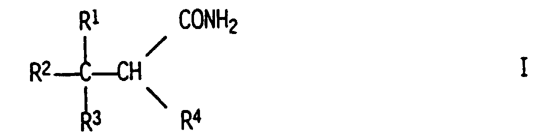 und die Aminoessigsäuren mit α-ständigem tertiären Kohlenwasserstoffrest oder deren Nitrile der Formel II: 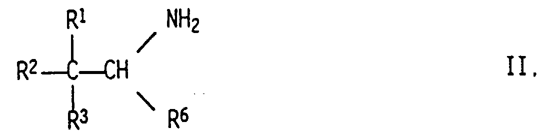 entsprechen, wobei R¹, R² und R³ gleiche oder verschiedene Kohlenwasserstoffreste bedeuten, jeweils zwei dieser Reste zudem mit dem quartären Kohlenstoffatom, das sie substituieren. einen Kohlenwasserstoffring bilden können, R⁴ eine Carboxyl-, Carbonester- oder Nitrilgruppe bedeutet und R⁶ für eine Carboxyl- oder Nitrilgruppe steht.

3. Verfahren nach Anspruch 2, dadurch gekennzeichnet das R¹, R² und R³ gleiche oder verschiedene Alkyl-, Alkenyl, Aryl-. Alkaryl- oder Aralkylreste mit bis zu 10 Kohlenstoffatomen bedeuten, jeweils zwei dieser Substituenten zudem mit dem quartären Kohlenstoffatom, das sie substituieren, einen Kohlenwasserstoffring mit 5 bis 12 Kohlenstoffatomen bilden können, R⁴ die Carboxylgruppe, die Nitrilgruppe oder eine Carbonestergruppe -COOR⁵ bedeutet, in der R⁵ einen Alkylrest mit 1 bis 4 Kohlenstoffatomen oder den Benzylrest bezeichnet, und R⁶ für eine Carboxyl- oder Nitrilgruppe Steht.

4. Verfahren nach einem der Ansprüche 1 bis 3, dadurch gekennzeichnet, daß man den Ausgangsstoff I mit 1.5 bis 4 Äquivalenten Natronlauge mischt, das Gemisch bei einer Temperatur von 0 bis 20°C mit 1.0 bis 1,2 Äquivalente Natriumhypochlorit-Lösung versetzt und dann das Reaktionsgemisch bei einer Temperatur von 20 bis 180°C nachreagieren läßt.

5. Verfahren nach einem der Ansprüche 1 bis 4, dadurch gekennzeichnet, daß man die Reaktion kontinuierlich oder diskontinuierlich durchführt.

6. Verfahren nach einem der Ansprüche 1 bis 5, dadurch gekennzeichnet, daß man das Reaktionsgemisch mit einer Mineralsäure neutralisiert und zur Trockne eindampft und die Aminoessigsäure mit α-ständigem tertiären Kohlenwasserstoffrest oder deren Nitril durch Extraktion des Eindampfrückstands mit einem selektiven Lösungsmittel von dem mineralsauren Salz abtrennt.

7. Verfahren nach Anspruch 6, dadurch gekennzeichnet, daß das selektive Lösungsmittel Methanol ist.

8. Verfahren nach einem der Ansprüche 1 bis 5, dadurch gekennzeichnet, daß man das Reaktionsgemisch mit einer Mineralsäure neutralisiert und das mineralsaure Salz durch ein Ionenaustausch- und/oder ein Membranverfahren von der Aminoessgsäure mit α-ständigem tertiären Kohlenwasserstoffrest oder deren Nitril abtrennt.

## Claims

1. A process for the preparation of an aminoacetic acid with a tertiary hydrocarbon radical in the α-position, or a nitrile thereof, characterized in that a malonic acid monoamide substituted in the α-position by a tertiary hydrocarbon radical, or an ester or nitrile thereof, is subjected to a Hofmann degradation.

2. A process according to claim 1, characterized in that the starting material has the formula I: 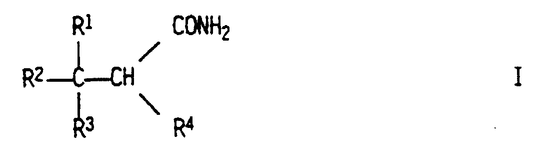 and the aminoacetic acid with a tertiary hydrocarbon radical in the α-position, or nitrile thereof, has the formula II: 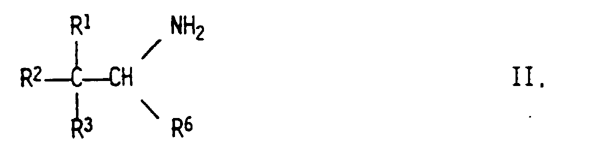 in which R¹, R² and R³ are identical or different hydrocarbon radicals, it also being possible for any two of these radicals to form a hydrocarbon ring with the quaternary carbon atom on which they are substituents, R⁴ is a carboxyl, carboxylic ester or nitrile group and R⁶ is a carboxyl or nitrile group.

3. A process according to claim 2, characterized in that R¹, R² and R³ are identical or different alkyl, alkenyl, aryl, alkaryl or aralkyl radicals having up to 10 carbon atoms, it also being possible for any two of these substituents to form a hydrocarbon ring having 5 to 12 carbon atoms with the quaternary carbon atom on which they are substituents, R⁴ is the carboxyl group, the nitrile group or a carboxylic ester group -COOR⁵, in which R⁵ is an alkyl radical having 1 to 4 carbon atoms or the benzyl radical, and R⁶ is a carboxyl or nitrile group.

4. A process according to any one of claims 1 to 3, characterized in that the starting material I is mixed with 1.5 to 4 equivalents of sodium hydroxide solution, 1.0 to 1.2 equivalents of sodium hypochlorite solution are added to the mixture at a temperature of 0 to 20°C and the reaction mixture is then allowed to react further at a temperature of 20 to 180°C.

5. A process according to any one of claims 1 to 4, characterized in that the reaction is carried out continuously or batchwise.

6. A process according to any one of claims 1 to 5, characterized in that the reaction mixture is neutralized with a mineral acid and evaporated to dryness and the aminoacetic acid with a tertiary hydrocarbon radical in the α-position, or nitrile thereof, is separated from the salt of the mineral acid by extraction of the evaporation residue with a selective solvent.

7. A process according to claim 6, characterized in that the selective solvent is methanol.

8. A process according to any one of claims 1 to 5, characterized in that the reaction mixture is neutralized with a mineral acid and the salt of the mineral acid is separated from the aminoacetic acid with a tertiary hydrocarbon radical in the α-position, or nitrile thereof, by an ion exchange technique and/or a membrane technique.

## Revendications

1. Procédé de fabrication d'acides aminoacétiques avec un radical hydrocarboné tertiaire en position α ou de leurs nitriles,
caractérisé en ce qu'
on soumet des monoamides de l'acide malonique substitués en position α par un radical hydrocarboné tertiaire, ou leurs esters ou nitriles, à une dégradation d'Hofmann.

2. Procédé selon la revendication 1,
caractérisé en ce que
les produits de départ correspondent à la formule I : 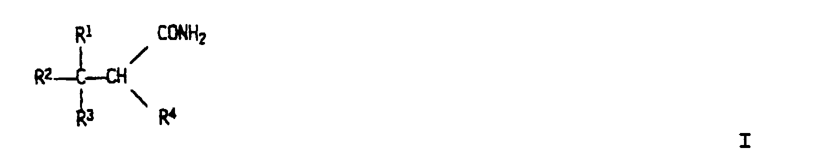 et les acides aminoacétiques avec un radical hydrocarboné tertiaire en position α ou leurs nitriles correspondent à la formule II : 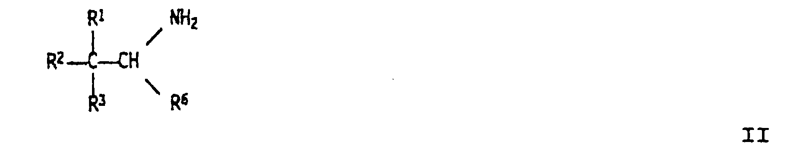 dans lesquelles R¹, R² et R³ représentent des radicaux hydrocarbonés identiques ou différents, deux de ces radicaux pouvant toujours former avec l'atome de carbone quaternaire qu'ils substituent un noyau hydrocarboné, R⁴ représente un groupe carboxyle, ester carbonique ou nitrile et R⁶ représente un groupe carboxyle ou nitrile.

3. Procédé selon la revendication 2,
caractérisé en ce que R¹, R² et R³ représentent des radicaux alkyle, alcényle, aryle, alcaryle ou aralkyle identiques ou différents ayant jusqu'à 10 atomes de carbone, deux de ces substituants pouvant toujours en outre former avec l'atome de carbone quaternaire qu'ils substituent un noyau hydrocarboné ayant de 5 à 12 atomes de carbone R⁴ représente le groupe carboxyle, le groupe nitrile ou un groupe ester carbonique -COOR⁵, dans lequel R⁵ représente un radical alkyle ayant de 1 à 4 atomes de carbone ou le radical benzyle, et R⁶ représente un groupe carboxyle ou nitrile.

4. Procédé selon l'une des revendications 1 à 3,
caractérisé en ce qu'
• on mélange le produit de départ I avec 1,5 à 4 équivalents de lessive de soude,
• on déplace le mélange à une température de 0 à 20°C avec 1,0 à 1,2 équivalent d'une solution d'hypochlorite de sodium, puis
• on fait réagir le mélange réactionnel à une température de 20 à 180°C.

5. Procédé selon l'une des revendications 1 à 4,
caractérisé en ce qu'
on conduit la réaction de façon continue ou discontinue.

6. Procédé selon l'une des revendications 1 à 5,
caractérisé en ce qu'
• on neutralise le mélange réactionnel avec un acide minéral,
• on le concentre jusqu'à siccité, et
• on sépare du sel minéral l'acide aminoacétique avec radical hydrocarboné tertiaire en position α ou son nitrile par extraction du résidu par évaporation avec un solvant sélectif.

7. Procédé selon la revendication 6,
caractérisé en ce que
le solvant sélectif est le méthanol.

8. Procédé selon l'une des revendications 1 à 5,
caractérisé en ce qu'
• on neutralise le mélange réactionnel avec un acide minéral, et
• on sépare le sel d'acide minéral, par un procédé d'échange d'ions ou un procédé à membrane, d'avec l'acide aminoacétique avec radical hydrocarboné tertiaire en position α ou son nitrile.
